(19) Europäisches Patentamt
European Patent Office
Office européen des brevets

(11) Publication number: **0 218 100**
**B1**

(12) # EUROPEAN PATENT SPECIFICATION

(45) Date of publication of patent specification: **29.11.89**

(51) Int. Cl.[4]: **C 07 C 43/15**, C 07 C 41/06, C 07 C 41/34

(21) Application number: **86112250.5**

(22) Date of filing: **04.09.86**

(54) **Conjugated alkadiene telomerization to organo-oxyalkadienes.**

(30) Priority: **09.09.85 US 773718**
**30.09.85 US 781590**

(43) Date of publication of application:
**15.04.87 Bulletin 87/16**

(45) Publication of the grant of the patent:
**29.11.89 Bulletin 89/48**

(84) Designated Contracting States:
**BE DE FR GB IT NL**

(56) References cited:
**FR-A-2 024 802**
**GB-A-2 074 156**
**US-A-4 219 677**

(73) Proprietor: **QUANTUM CHEMICAL CORPORATION (a Virginia corp.)**
**99 Park Avenue**
**New York, NY 10016 (US)**

(72) Inventor: **Hanes, Ronnie M.**
**5817 Mt. Vernon Drive**
**Milford Ohio (US)**

(74) Representative: **Patentanwälte Grünecker, Kinkeldey, Stockmair & Partner**
**Maximilianstrasse 58**
**D-8000 München 22 (DE)**

**EP 0 218 100 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention is directed to methods for the telomerization of a conjugated alkadiene with an alkanol in the presence of a catalyst to produce an alkoxyalkadiene.

The telomerization of conjugated alkadienes such as butadiene with alkanols in the presence of a palladium-phosphine catalyst is a convenient route for the preparation of 8-methoxy-1,6-octadiene. Because of the high cost of palladium catalysts, any industrial process employing catalysts of this type requires recycling of the catalyst in order to minimize the cost of production. Accordingly, catalyst recovery and recycle is of primary concern along with selectivity, yields and speed of reaction in the selection of a catalyst for telomerization reactions of this type. Although palladium-phosphine type catalysts are effective for promoting these types of telomerization reactions after recovering and recycling the catalyst for subsequent reactions, it has been observed that the activity of the catalyst drops off significantly. Additionally, prior to recovering and recycling the catalyst the various products of the telomerization reaction must be separated by a process which does not substantially damage or deactivate the catalyst or cause a loss of the catalyst. The prior art method of recovering the telomerization product generally comprised distillation of the reaction products to remove them from the catalyst and resulted in catalyst loss and/or deactivation.

GB—A—2074156 discloses a process for preparing n-octadienol comprising reacting butadiene with water in an aqueous sulpholane solution that has a water:sulpholane weight ratio in the range of 20:80 to 70:30 in the presence of a catalyst comprising (A) palladium or a palladium compound, (B) a monodentate phosphine of a specified formula, and (C) a monodentate tertiary amine.

US—A—4219677 discloses a process for telomerizing dienes which comprises the step of reacting a diene with a non-aqueous telomerizing compound containing at least one mobile hydrogen atom, said telomerizing agent being selected from an alcohol or phenol, in the presence of a catalytic system comprising a specified water-soluble sulfonated triphenylphosphine compound and a palladium compound.

Said processes, however, do not result in high selectivities of the final product.

It is the object of the present invention to provide a method for the telomerization of a conjugated alkadiene with an alkanol to produce an alkoxyalkadiene with high selectivity.

Said object is achieved by a method for the telomerization of a conjugated alkadiene having 4 to 6 carbon atoms with an alkanol to produce an alkoxyalkadiene by reacting said conjugated alkadiene and said alkanol with a catalytically effective amount of a catalyst compound comprising

$$[PdR^1] \quad [YR^2_y]_2 \qquad (I)$$

wherein $R^1$ is an organo acyclic or cyclic carboxylate group having from 1 to 10 carbon atoms and $R^2$ is an organo straight chain, branched chain or cyclic radical having from 1 to 10 carbon atoms, Y is phosphorous, arsenic or antimony, y is the valence of Y, wherein said alkanol is used in an excess amount of the stoichiometric amount required for the telomerization, the ligand:Pd ratio is maintained at from 10:1 to 1:1 during the reaction, said reaction being conducted in the substantial absence of oxygen and water and in the presence of a high boiling organic solvent for the catalyst, the boiling point of said solvent being greater than the boiling point of said alkoxy-alkadiene;

separating by distillation said alkoxyalkadiene from said catalyst without addition of water and recycling said catalyst for the further telomerization of said conjugated alkadiene with said alkanol.

Said object is also achieved by a method for the telomerization of a conjugated alkadiene having 4 to 6 carbon atoms with an alkanol to produce an alkoxyalkadiene by reacting said conjugated alkadiene with said alkanol in the presence of a catalytically effective amount of a catalyst compound comprising

$$[PdR^1] \quad [R^2_xY(R^3Z)_y] \qquad (II)$$

wherein $R^1$ is an organo acyclic or cyclic carboxylate group having from 1 to 10 carbon atoms, $R^2$ is a cyclic or acyclic hydrocarbon group having up to 10 carbon atoms, Y is phosphorous, antimony, arsenic or nitrogen, x + y is the valence of Y wherein y is equal to or greater than 1, $R^3$ is a cyclic or acyclic hydrocarbon group having up to 6 carbon atoms or naphthyl, Z is a hydrophilic group selected from $—SO_3Na$, $—NH_2$, —COOH and quaternary ammonium groups, said reaction being conducted in the substantial absence of oxygen and water and in the presence of a polar solvent for the catalyst to obtain a telomer and extracting said telomer with a hydrocarbon solvent.

According to the method of the first embodiment of the present invention, the solvent is a high boiling solvent such that the alkoxyalkadiene product can be distilled from the reaction mixture (which includes the catalyst) without having any substantial adverse affect on the catalyst. Vacuum fractional distillation, in this embodiment, is especially suitable for separating the alkoxyalkadiene from the solution of the catalyst

without adversely affecting the catalyst although flash distillation and atmospheric distillation methods may be used.

In the catalyst of the general formula (I) $R^1$ is an organo acyclic or cyclic carboxylate group having from 1 to 10 carbon atoms and especially a lower alkyl straight chain or branched chain carboxylate having from 1 to 4 carbon atoms, especially the acetate group. $R^2$ is an organo straight chain, branched chain or cyclic radical having from 1 to 10 carbon atoms and especially is either a phenyl group or a lower alkyl group having from one to 4 carbon atoms.

In the formula (I) the moiety Y is either phosphorous, arsenic or antimony wherein y is the valence of Y.

In the above formula (I) ligands $[YR^2_y]$ may comprise:

Tributylphosphine
Tripentylphosphine
Trihexylphosphine
Tritolylphosphine
Triphenylphosphine
Tris(methoxyphenyl)phosphine
Triphenylphosphite
Tritolylphosphite
Trioctylphosphine

and the arsine and stibine homologs thereof.

The $R^2$ radical preferably comprises a phenyl group. The stibines, arsines or phosphines containing alkyl groups are more susceptible to oxidation.

The palladium component of the catalyst complex herein can also be zero-valent palladium, a palladium-containing composition which will provide zerovalent palladium, i.e., will undergo reduction, under the conditions of the reaction and/or a palladium (II) salt, with or without the additional presence of a reducing agent such as an alkali metal alkoxide, an alkali metal acetate and/or an alkali metal borohydride. Among such palladium-containing compositions are included palladium (II) acetate, palladium (II) formate, palladium (II) octanoate or palladium (II) propionate.

The telomerization reaction is conducted in the substantial absence of oxygen and water and especially under conditions in which oxygen is excluded to minimize the amount of catalyst ligand that must be replaced due to the oxidation of the stibene, arsine or phosphine moieties in the ligand. It has been discovered that the presence of even small quantities of oxygen had a deleterious affect in that oxygen causes ligand removal from the catalyst and the decomposition of the catalyst.

One of the catalysts employed according to the invention and falling within the scope of the above formula (I) is palladium acetate bis(triphenylphosphine) $[Pd(OAc)_2]$ $[PPh_3]_2$. This is one of the preferred catalysts.

In the above catalyst compound, $[PdR^1]$ is referred to herein as the palladium component of the catalyst and $[YR^2_y]$ as the ligand. When the catalyst is employed in the method of the present invention, the palladium and the ligand dissociate so that under reaction conditions the ratio of palladium to the ligand varies. Accordingly, by the addition of the ligand or palladium to the reaction mixture containing the catalyst or by the adjustment of the reaction conditions (e.g., exclusion of oxygen) the ratio of the ligand to palladium of the catalyst compound in the reaction millieu is controlled to anywhere from 10:1 to 1:1 and preferably a ratio of from 5:1 to 2:1 on a molar basis.

An essential feature of this embodiment of the present invention is to employ a solvent for the catalyst in which the boiling point of the solvent is higher than that of the alkoxyalkadiene product that is produced as a result of the telomerization reaction. Generally, any solvent may be employed that is inert to the reactants and which does not have an adverse affect on the reaction. Solvents falling into this category may have a normal boiling point up to 300°C and generally comprise those solvents having a normal boiling point at least 20°C higher than the alkoxyalkadiene that is produced up to 300°C. Solvents especially suitable in this regard comprise tetraglyme (dimethyl ether of tetraethylene glycol); dimethyl phthalate; dibutyl phthalate; dioctyl phthalate; methyl oleate; diphenyl oxide; diethylene glycol; diethylene glycol mono-sec butyl ether acetate (butyl Carbitol acetate); diethylene glycol monobutyl ether (butyl ethyl Cellosolve); di-octyl azelate; methyl benzoate; butyrolactone; and NMP (N-methylpyrrolidone).

After the alkoxyalkadiene is formed, it is separated from the solution of the catalyst and the solvent by distillation, such as by vacuum fractional distillation, flash distillation or atmospheric distillation. The distillation method is dictated by the temperature at which by distillation is to be conducted. These temperatures preferably are from 80°C to 110°C and are selected so as to avoid any adverse affects on the catalyst. Vacuum fractional distillation is the preferred method of separating the alkoxyalkadiene. Standard vacuum distillation apparatus and methods are employed. It has been found that by separating the alkoxyalkadiene form the catalyst in this manner, the catalyst is not adversely affected by the separation process and may be recycled for further use in the method of the present invention.

It has also been found that by substantially excluding oxygen from the telomerization reaction and by employing the aforesaid class of organic solvents for the catalyst that the catalyst can be separated by distillation and recycled to the telomerization reaction in excess of 15 times without any substantial loss of catalyst activity i.e., selectivity and yield of the alkoxyalkadiene. Furthermore, between 50,000 to 60,000 moles of the alkoxyalkadiene may be produced for each mole of catalyst employed in the method of the

present invention.

One of the advantages of the method of the present invention is that about 90% selectivity to methoxyoctadiene is obtained at butadiene conversions of up to about 80%. Selectivities of from about 85% to about 95% at conversions of butadiene up to about 100% can be obtained with the catalyst and the method of the present invention when the telomerization reaction is conducted at temperatures from 60°C to 80°C for a period of time from 15 to 30 min. It has also been found that conversions and selectivities obtained in the telomerization reaction are independent of pressure.

It has also been discovered that selectivity and yields of the telomerization reaction may be optimized at 65°C to 75°C at autogenous pressures up to 1379 kPa (200 psig) in an inert atmosphere such as an atmosphere comprising nitrogen, one of the rare gases selected from group VIII A of the Periodic Table of Elements and various mixtures thereof.

In one preferred embodiment, it has been discovered that butadiene is telomerized with a lower alkanol such as methanol in the presence of a palladium acetate triphenylphosphine catalyst where the ratio of the triphenylphosphine to palladium acetate is from 3 to 5. A high boiling solvent such as tetraglyme and the equivalents thereof are also employed and the reaction is run at a temperature of about 70°C to obtain high yields, selectivities and conversions to methoxyoctadiene. High selectivities to 8-methoxy-1,6-octadiene have been obtained in this regard with smaller amounts of 3-methoxy-1,7-octadiene also being produced, the ratio of 8-methoxy-1,6-octadiene to 3-methoxy-1,7-octadiene being greater than 4:1 on a molar basis. Optimum ratios of butadiene to alkanol and especially methanol were obtained by adjusting the molar ratios of butadiene to alkanol (i.e., methanol) to about 1 to about 1.2. By employing the method of the present invention in which oxygen is excluded from the reaction and the high boiling solvent is used for the catalyst so that the methoxyoctadiene may be separated by vacuum distillation the catalyst may be successfully reused in excess of 15 times and from 50,000 to 60,000 moles of methoxyoctadiene may be produced for one mole of the palladium catalyst.

It has been found that during the successive recycling of the catalyst by the method of the present invention that even with substantial exclusion of oxygen from the process that the ligand is diminished and that in subsequent recycles of the catalyst thus recovered, small amounts of the ligand are added to the catalyst to replenish that proportion of ligand that is lost. Anywhere from 1% to 10% of the theoretical amount of ligand is added to each successive recycle of the catalyst in this respect when proper precautions are taken to exclude oxygen from the reaction.

According to the method of the second embodiment of the present invention a catalyst compound of the general formula (II) is used. In the general formula (II)

$R^1$ is an organo acyclic or cyclic carboxylate group having from 1 to 10 carbon atoms, especially the acetate group. $R^1$ more particularly is a straight chain, branched chain or cyclic radical having from 1 to 10 carbon atoms and especially is either a phenyl group or a lower alkyl group having from 1 to 4 carbon atoms.

The palladium component of the catalyst complex [$PdR^1$] can also be zero-valent palladium, a palladium-containing composition which will provide zerovalent palladium, i.e., will undergo reduction, under the conditions of the reaction and/or a palladium (II) salt, with or without the additional presence of a reducing agent such as an alkali metal alkoxide, an alkali metal acetate and/or an alkali metal borohydride. Among such palladium-containing compositions are included palladium (II) acetate, palladium (II) formate, palladium (II) octanoate and palladium (II) propionate.

In the formula (II) the ligand

$$[R^2_xY(R^3Z)_y]$$

comprises compounds wherein:

(a) $R^2$ is a cyclic or acyclic hydrocarbon group having up to 10 carbon atoms, such as phenyl, benzyl and naphthyl,

(b) Y is phosphorous, antimony, arsenic or nitrogen,

(c) x+y is the valence of Y where Y is equal to or greater than 1,

(d) $R^3$ is a cyclic or acyclic hydrocarbon group having up to about 6 carbon atoms such as phenyl, ethylene, or benzyl or naphthyl,

(e) Z is a hydrophylic group selected from $—SO_3Na$, $—NH_2$, —COOH, or a quaternary ammonium group such as $N^+Me_3NO_3^-$.

Ligands that are especially suitable comprise:

Tris(aminophenyl)phosphine

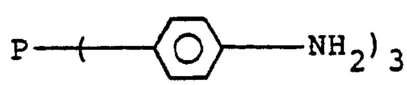

Bis(aminophenyl)phenylphosphine

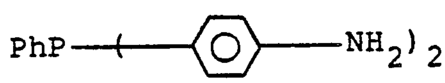

Aminophenyldiphenylphosphine

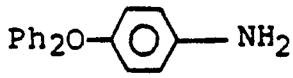

Sodium diphenylphosphinobenzene-m-sulfonate

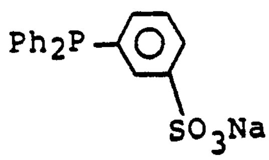

Trisodium-tris(m-sulphophenyl)phosphine

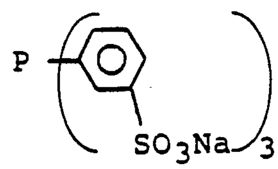

Trisodium-tris(m-sulphophenyl)arsine

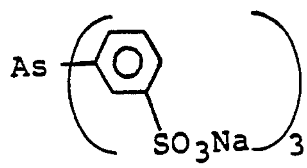

2-Diphenylphosphinoethyl trimethylammonium nitrate (a.k.a. amphos nitrate)

$$[Ph_2PCH_2CH_2N{+}Me_3][NO_3]$$

Tris(carboxyphenyl)phosphine

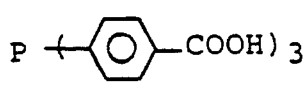

Bis(carboxyphenyl)phenylphosphine

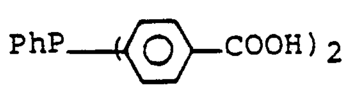

Carboxyphenyldiphenylphosphine

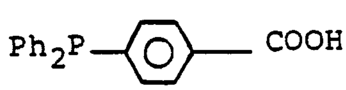

The various arsine and stibine homologs of the foregoing ligands may also be employed. The ratio of the ligand to palladium is from 2:1 to 50:1 on a molar basis. The ligand component is added to the catalyst in subsequent telomerization reactions because of loss for example due to oxidation or product separation.

The solvent for the catalyst is a polar organic solvent such that the reaction products may be separated from the solution of the catalyst by extraction with a solvent such as a hydrocarbon that is substantially immiscible in the polar solvent and which acts as a solvent for the reaction products. By employing this method of the present invention deactivation of the catalyst during the telomerization reaction is avoided by substantially eliminating contact of the reaction mass with oxygen and further, by using a solvent extraction process that minimizes exposure of the reaction products and the catalysts to air catalyst deactivation is further minimized.

One of the essential features of this embodiment of the present invention is to employ a solvent for the catalyst which is polar such as N,N-dimethylformamide (DMF) or sulfolane. Generally, the polar solvents useful in this respect comprise the N-substituted amides in which the hydrogen of the amido nitrogen is substituted by a hydrocarbyl group e.g. 1-methyl-pyrrolidin-2-one; N,N-dimethylacetamide; N,N-diethylacetamide; N-methylpiperidone; 1,5-dimethylpyrrolidin-2-one; 1-benzyl-pyrrolidin-2-one; N,N-dimethyl-

propionamide; hexamethylenephosphoric triamide and similar amides that are liquid at room temperature; sulfolane and glycols such as ethylene glycol, propylene glycol or butylene glycol; polyglycols such as polyethylene glycol, polypropylene glycol, polybutylene glycol and mixtures thereof; mono-lower alkyl ethers of alkylene glycols and polyalkylene glycols e.g. methyl ethers of ethylene glycol, propylene glycol, and di-, tri- and tetra-ethylene glycols: dimethylsulfoxide (DMSO) and mixtures thereof. Those solvents having a high dielectric constant are the most preferred polar solvents.

The use of a polar solvent facilitates extraction of the product and recovery and recycling of the catalyst. In the present process, it is possible to extract the telomer product from the reaction mixture by using a hydrocarbon which is a solvent for the telomer such as hexane. Any similar hydrocarbon solvent may be employed for example, n-pentane, n-heptane, n-octane, n-nonane, iso-octane, cyclohexane, methylcyclohexane or mixtures thereof. The hydrocarbon solvents therefore comprise any acyclic or cyclic saturated hydrocarbon having up to 10 carbon atoms. Where the hydrocarbons may be gaseous at room temperature, the separation is conducted at elevated pressures at temperatures less than the critical temperature of the hydrocarbon. Hexane is one of the preferred organic solvents for extraction of the telomer. The extraction of the product leaves the catalyst in the polar solvent for recycling and thus avoids exposing the catalyst to fractional distillation processes typically used for product-catalyst separation. Multiple extractions can be used since in some cases the telomer may also be soluble to some small extent in the polar solvent. Co-current or counter current extraction processes are also utilized for the separation of the catalyst and the telomer. The polar and the hydrocarbon solvents are employed in combination with one another as noted above and are selected so as to form an immiscible pair which is within the ordinary skill of the art.

The reaction may be conducted, in this embodiment, at pressures from atmospheric pressure up to about 1379 kPa (200 psig) and at temperatures from 60°C to 80°C and especially from 65°C to 75°C.

Independent of which embodiment of the method of the present invention is practiced it has been discovered that the molar ratio of the alkadiene to the alkanol influences the yield and selectivity of the reaction and that an excess of the alkanol is required to maximize such yields and selectivities. By an excess it is meant that the alkanol is present in more than a stoichiometric amount (i.e., greater than 1 mole of alkanol to 2 moles of conjugated alkadiene). The molar ratio of the aforesaid alkanol to the aforesaid alkadiene is from about 0.6:1 to about 3:1 and preferably from 0.75:1 to 2.5:1.

The alkanol specifically may comprise a compound having from 1 to 10 carbon atoms and may be a straight chain, branched chain or cyclic compound having at least one hydroxy group. Organic hydroxy compounds in this respect may for example comprise methanol, ethanol, 1-propanol, 2-propanol, 2-butanol, hydroxycyclohexane and hydroxycyclopentane.

The conjugated alkadiene has anywhere from 4 to 6 carbon atoms and comprises for example butadiene, isoprene, chloroprene, piperylene, and 1,3-pentadiene.

The following examples are given to illustrate the invention.

Example 1

A 2 l 316 stainless steel, stirred reactor was charged with 0.16 g (0.72 mmole) $Pd(OAc)_2$, 150 ml (150.5 g or 677 mmole) tetraglyme, 225 ml (178 g or 5.565 mmole) methanol, 0.942 g (3.59 mmole) triphenylphosphine ($PPh_3$), 5.06 g (46.8 mmole) anisole, and was purged 3 times with nitrogen. Anisole is an internal standard for glc analysis. The reactor was charged with 265 g of butadiene (4900 mmole) and the reactor and its contents heated to 75°C were held for 30 min at this temperature and cooled overnight.

The contents of the reactor were then discharged under inert atmosphere and distilled by vacuum distillation at 3.3 kPa (25 mm Hg) pressure and 1.392.0 mmoles of 8-methoxy-1,6-octadiene were obtained along with 375.6 mmoles of 3-methoxy-1,7-octadiene.

The bottoms from the distillation process contained the palladium acetate triphenylphosphine catalyst and were recycled for subsequent telomerization of butadiene and methanol in substantially the same way as described above with the exception that additional triphenylphosphine ligand was added after each recycle of the catalyst and telomerization reaction. The triphenylphosphine added was anywhere from about 0.4 mmole to about 1.2 mmole in 12 recycles depending upon the amount of ligand that was determined to have been lost and substantially the same results were obtained as set forth in the above paragraph.

When the catalyst was recycled in subsequent reactions, anisole was not added to the reactor.

Example 2

The procedure of Example 1 was substantially repeated in seven different telomerization reactions to study the affect of the methanol to butadiene ratio on the telomerization reaction. The results are shown in Table 1.

TABLE I
METHANOL:BUTADIENE RATIO[a]

| M:B Ratio[b] | Ethers[c] (mmoles) | Selectivity (%)[c] Ethers | Octatriene |
|---|---|---|---|
| 0.4 | 51 | 75% | 5% |
| 0.653 | 78 | 81 | 6 |
| 0.894 | 112 | 87 | 6 |
| 1.115 | 128 | 89 | 6.5 |
| 1.35 | 140 | 96 | 4 |
| 1.58 | 102 | 94 | 4 |
| 1.83 | 62 | 95 | 5 |

[a]0.287 mmol $Pd(OAc)_2$, 0.574 mmol $PPh_3$, 30 ml dimethylphthalate, 5 ml anisole, total volume = 165 ml, 60°C.
[b]Methanol:butazdiene molar ratio (as charged to reactor). Total of 130 ml total volume of methanol and liquid butadiene.
[c]Based on 15 min —0 min sample results.

The methanol:butadiene stoichiometry is 0.5 and it would therefore be expected that this would be the optimum ratio of these two components in order to obtain methoxy-octadiene. The data in this table, therefore, indicates that the selectivity and ether yield are maximized at a methanol:butadiene molar ratio greater than 1:1 which is unexpected.

Example 3

The telomerization of butadiene to methoxyoctadiene was also studied in a continuous-stirred tank reactor. The preformed complex, $Pd(OAc)_2(PPh_3)_2$, was used as catalyst in dimethyl phthalate-methanol solution. Runs were assumed to have achieved steady-state when two criteria were met: (1) ether production was constant over at least the final three space-times (chemical steady-state) and (2) the dimethyl phthalate pumped in and out of the reactor balanced (physical steady-state). All such runs met both criteria).

In initial studies on temperature effects, the highest selectivity to methoxyoctadiene (86%) was obtained at 70°C; conversion increased from 16% to 80% as temperature was increased from 60°C to 80°C (Table II). Since ether yields were equivalent at 70°C and 80°C (64%) further studies were conducted at 70°C.

At a ligand to palladium ratio of 2, catalyst decomposition to palladium metal was observed in the reactor and 54% conversion was obtained. At higher ratios the catalyst was stable but conversion decreased above a ratio of 5. Maximum selectivity and conversion were attained at a ratio of 3.

The affect of reactant steady-state concentration on reaction rate was also determined in the continuous reactor (Table III). The reaction rates were high in most cases with a maximum rate observed at 4.0*M* butadiene and 3.14*M* methanol. Butadiene conversion varied from 40 to 55% and ether selectivity varied from 91 to 94% in these runs. These data indicate that as the rate increased, ether selectivity also increased.

TABLE II
BUTADIENE TELOMERIZATION
TEMPERATURE EFFECT AT STEADY STATE[1]

| | Temp., °C | | |
|---|---|---|---|
| | 60 | 70 | 80 |
| Ether Selectivity (%)[2] | 74 | 86 | 79 |
| Product Yield (%)[2] | 16 | 74 | 80 |

[1]Space Time = 15 min, [Pd] = $1.9 \times 10^{-3}M$, $PPh_3$:Pd = 3.
[2]Based on products detected by glc techniques.

TABLE III
BUTADIENE TELOMERIZATION
REACTANT CONCENTRATION EFFECT ON RATE[1]

| Steady-State Concentration: Butadiene M | Methanol (M) | Observed Ethers Space Time Yield ($g/cm^3/h$) [$lbs/ft^3/h$] |
|---|---|---|
| 1.60 | 6.42 | 0.46 [29] |
| 1.90 | 6.45 | 0.32 [20] |
| 2.35 | 3.92 | 0.86 [54] |
| 2.40 | 5.60 | 0.90 [56] |
| 2.49 | 6.43 | 0.51 [32] |
| 2.67 | 7.90 | 0.66 [41] |
| 2.93 | 2.38 | 0.67 [42] |
| 3.54 | 5.63 | 0.86 [54] |
| 4.00 | 3.14 | 1.06 [66] |

[1][Pd] = $1.9 \times 10^{-3}M$, $PPh_3$/Pd = 3, 70°C, 15 min space time, 150 ml volume.

Example 4

The telomerization reaction was also run in a 450 ml plug-flow reactor consisting of 23.6 m (77 ft-5 inches) of 2.54 m ($\frac{1}{4}$ inch) I.D. stainless steel tubing immersed in a 73°C water/ethylene glycol bath. To this reactor were fed three streams: a catalyst solution of 0.0063*M* $Pd(OAc)_2(PPh_3)_2$ and 0.0063*M* $PPh_3$ in methyl benzoate (containing 9.1 volume percent methanol), fed at an average of 3.4 ml/min; methanol, fed at an average of 2.3 ml/min; and butadiene, fed at an average of 5.6 ml/min. The reactor was maintained at 65.5 kPa (95 psig) in order to main a liquid phase in the reactor. Over an eleven hour period with an average residence time of reactants in the reactor of 40.1 min the average yield of 8-methoxy-1,6-octadiene was 57.4% (based on butadiene fed) and 2.5 moles 8-methoxy-1,6-octadiene/l reactor/h were obtained. Average selectivity to 8-methoxy-1,6-octadiene was 81.1% (based on butadiene converted). No catalyst decomposition was observed.

Comparative Example 1

To a pyrex tube 0.0099 g (0.045 mmoles) palladium acetate, 0.049 g (0.134 mmoles) sodium diphenylphosphinobenzene-m-sulfonate, 8 ml methanol and 8 ml butadiene were added. The tube was sealed and placed in a 75°C oil bath for 1.5 h. Very little butadiene gas remained in the tube and a bright yellow solution

with no precipitate was recovered. The product was analyzed by glc on a Silar 10C column and contained 5.6 g, 40 mmoles of methoxyoctadiene.

Example 5

Butadiene telomerization with methanol, recycle of catalyst.

To a pyrex tube 0.0103 g palladium acetate, 0.0500 g of the same ligand employed in Comparative Example 1, 8 ml methanol, 5 ml sulfolane and 10 ml butadiene were added. The tube was sealed and placed in a 70°C oil bath for 1 h. The tube was cooled, vented and a sample taken for glc analysis. The remainder was extracted with 25 ml hexane and the sulfolane layer returned to the tube with 8 ml methanol and 10 ml butadiene was added. The tube was placed in a 70°C oil bath for 1.5 h. The product solution was extracted twice with 25 ml hexane, then 8 ml methanol and 10 ml butadiene were added to the sulfolane layer and the tube placed in a 70°C oil bath for 1.5 h. The product obtained after each one of the telomerizations was analyzed in the same manner as set forth in Comparative Example 1 and each contained 7.5 g or 50 mmoles methoxyoctadiene.

Example 6

Butadiene telomerization

To a pyrex tube 0.0202 g palladium acetate, 0.1033 g of the ligand employed in Comparative Example 1, 16 ml methanol, 10 ml sulfolane and 20 ml butadiene were added. The tube was sealed and placed in a 70°C oil bath for 1.5 h. The product solution was extracted twice with 25 ml portions of hexane. To the sulfolane layer 16 ml methanol and 20 ml butadiene were added. The tube was sealed and placed in a 70°C oil bath for 2 h. The product solution (55 ml) was extracted twice with 75 ml portions of hexane. To the sulfolane layer 8 ml methanol and 20 ml butadiene were added. The tube was returned to a 75°C oil bath for 2 h. The product solution was extracted with 75 ml hexane and the sulfolane layer analyzed by atomic absorption and 0.068 g Pd (75% of original) was found.

Hexane was removed from the telomer extracted in each run and the residue was weighed. In the first run 1.5 g was recovered, in the second 14.9 g and in the third 17.5 g. These residues were analyzed and were found to contain 29.0 g (207 mmoles) methoxyoctadiene.

Example 7

To a pyrex tube 0.206 g palladium acetate, 0.1030 g of the ligand employed in Comparative Example 1, 16 ml methanol, 10 ml DMF and 20 ml butadiene were added. The tube was placed in a 70°C oil bath for 2 h. The product solution was extracted twice with 75 ml hexane. The DMF solution was returned to the tube with 8 ml methanol and 20 ml butadiene. The tube was placed in a 70°C oil bath for 2 h and the product solution extracted twice with 75 ml of hexane in each extraction. The telomer obtained was analyzed by glc in the same manner as in Example 5 and contained 28 g (200 mmoles) total methoxyoctadiene.

The telomers obtained according to the method of the invention may be carbonylated to form unsaturated esters by art known methods. The esters obtained may be hydrogenated and used as lubricants, plasticizers or functional fluids or may be hydrolyzed to form an acid having unsaturated groups. The acid obtained may be incorporated into polyesters manufactured from phthalic anhydride, glycols and maleic anhydride and which are subsequently cross-linked with styrene, all of which is known in the art. The unsaturated acid obtained provides a site along the polyester chain for cross-linking with styrene or equivalent monomers.

**Claims**

1. A method for the telomerization of a conjugated alkadiene having 4 to 6 carbon atoms with an alkanol to produce an alkoxyalkadiene by reacting said conjugated alkadiene and said alkanol with a catalytically effective amount of a catalyst compound comprising

$$[PdR^1] \quad [YR^2_y]_2 \qquad (I)$$

wherein $R^1$ is an organo acyclic or cyclic carboxylate group having from 1 to 10 carbon atoms and $R^2$ is an organo straight chain, branched chain or cyclic radical having from 1 to 10 carbon atoms, Y is phosphorous, arsenic or antimony, y is the valence of Y, wherein said alkanol is used in an excess amount of the stoichiometric amount required for the telomerization, the ligand:Pd ratio is maintained at from 10:1 to 1:1 during the reaction, said reaction being conducted in the substantial absence of oxygen and water and in the presence of a high boiling organic solvent for the catalyst, the boiling point of said solvent being greater than the boiling point of said alkoxy-alkadiene;

separating by distillation said alkoxyalkadiene from said catalyst without addition of water and recycling said catalyst for the further telomerization of said conjugated alkadiene with said alkanol.

2. The method of claim 1 wherein said conjugated alkadiene is selected from butadiene, isoprene, chloroprene, piperylene and 1,3-pentadiene.

3. The method of claim 1 or 2 wherein said ligand:Pd ratio is maintained at from 5:1 to 2:1.

4. The method of any of claims 1 to 3 wherein said alkanol is selected from 1 to 10 carbon atom straight chain, branched chain or cyclic compounds having at least one hydroxy group.

5. The method of claim 4 wherein said alkanol is methanol.

6. The method of claim 1 wherein $R^1$ is a straight chain or branched chain carboxylate having from 1 to 4 carbon atoms.

7. The method of claim 1 wherein $R^2$ is a phenyl group or an alkyl group having from 1 to 4 carbon atoms.

8. The method of any of claims 1 to 7 wherein said reaction is conducted at a temperature of from 65°C to 75°C at autogenous pressures up to 1379 kPa (200 psig) in an inert atmosphere.

9. A method for the telomerization of a conjugated alkadiene having 4 to 6 carbon atoms with an alkanol to produce an alkoxyalkadiene by reacting said conjugated alkadiene with said alkanol in the presence of a catalytically effective amount of a catalyst compound comprising

$$[PdR^1]\quad [R^2_xY(R^3Z)_y] \qquad (II)$$

wherein $R^1$ is an organo acyclic or cyclic carboxylate group having from 1 to 10 carbon atoms, $R^2$ is a cyclic or acyclic hydrocarbon group having up to 10 carbon atoms, Y is phosphorous, antimony, arsenic or nitrogen, x + y is the valence of Y wherein y is equal to or greater than 1, $R^3$ is a cyclic or acyclic hydrocarbon group having up to 6 carbon atoms, benzyl or naphthyl, Z is a hydrophilic group selected from $—SO_3Na$, $—NH_2$, —COOH and quaternary ammonium groups, said reaction being conducted in the substantial absence of oxygen and water and in the presence of a polar solvent for the catalyst to obtain a telomer and extracting said telomer with a hydrocarbon solvent.

10. The method of claim 9 wherein said conjugated alkadiene is selected from butadiene, isoprene, chloroprene, piperylene and 1,3-pentadiene.

11. The method of claim 9 or 10 wherein said alkanol is selected from straight chain, branched chain or cyclic compounds having up to 10 carbon atoms and at least one hydroxy group.

12. The method of claim 11 wherein said alkanol is methanol.

13. The method of claim 9 wherein $R^1$ comprises a phenyl group or a lower alkyl group having 1 to 4 carbon atoms.

14. The method of any of claims 9 to 13 wherein said reaction is conducted at a temperature of from 60°C to 80°C at autogenous pressures up to about 1379 kPa (200 psig) in a non-oxidizing atmosphere under anhydrous conditions.

15. The method of any of claims 9 to 14 wherein said polar solvent is selected from N-substituted amides, glycols, dimethyl sulfoxide and sulfolane and said hydrocarbon solvents contain up to 10 carbon atoms and are selected from acyclic or cyclic saturated hydrocarbons.

16. The method of any of claims 9 to 15 wherein said catalyst comprises a complex of palladium acetate and sodium diphenylphosphinobenzene-m-sulfonate.

**Patentansprüche**

1. Verfahren zur Telomerisation eines konjugierten Alkadiens mit 4 bis 6 Kohlenstoffatomen mit einem Alkanol zur Herstellung eines Alkoxyalkadiens durch Umsetzung des konjugierten Alkadiens und des Alkanols mit einer katalytisch wirksamen Mengen einer Katalysatorverbindung, umfassend

$$[PdR^1]\quad [YR^2_y]_2 \qquad (I)$$

worin $R^1$ eine organische acyclische oder cyclische Carboxylatgruppe mit 1 bis 10 Kohlenstoffatomen ist und $R^2$ ein organischer, geradkettiger, verzweigter oder cyclischer Rest mit 1 bis 10 Kohlenstoffatomen ist, Y Phosphor, Arsen oder Antimon ist, y die Wertigkeit von Y ist, worin das Alkanol in einer Überschußmenge der für die Telomerisation erforderlichen stöchiometrischen Menge verwendet wird, das Ligand:Pd-Verhältnis bei 10:1 bis 1:1 während der Reaktion gehalten wird, die Reaktion in wesentlicher Abwesenheit von Sauerstoff und Wasser und in Gegenwart eines hochsiedenden organischen Lösungsmittels für den Katalysator durchgeführt wird, wobei der Siedepunkt des Lösungsmittels größer als der Siedepunkt des Alkoxyalkadiens ist;

Trennen des Alkoxyalkadiens von dem Katalysator durch Destillation ohne Zugabe von Wasser und Rückführen des Katalysators für die weitere Telomerisation des konjugierten Alkadiens mit dem Alkanol.

2. Verfahren nach Anspruch 1, worin das konjugierte Alkadien aus Butadien, Isopren, Chlorpren, Piperylen und 1,3-Pentadien gewählt wird.

3. Verfahren nach Anspruch 1 oder 2, worin das Ligand:Pd-Verhältnis bei 5:1 bis 2:1 gehalten wird.

4. Verfahren nach einem der Ansprüche 1 bis 3, worin das Alkanol aus 1 bis 10 Kohlenstoffatome

aufweisenden geradkettigen, verzweigten oder cyclischen Verbindungen mit wenigstens einer Hydroxygruppe gewählt wird.

5. Verfahren nach Anspruch 4, worin das Alkanol Methanol ist.

6. Verfahren nach Anspruch 1, worin $R^1$ ein geradkettiges oder verzweigtes Carboxylat mit 1 bis 4 Kohlenstoffatomen ist.

7. Verfahren nach Anspruch 1, worin $R^2$ eine Phenylgruppe oder eine Alkylgruppe mit 1 bis 4 Kohlenstoffatomen ist.

8. Verfahren nach einem der Ansprüche 1 bis 7, worin die Umsetzung bei einer Temperatur von 65°C bis 75°C bei autogenen Drucken bis zu 1379 kPa (200 psig) in einer inerten Atmosphäre durchgeführt wird.

9. Verfahren zur Telomerisation eines konjugierten Alkadiens mit 4 bis 6 Kohlenstoffatomen mit einem Alkanol zur Herstellung eines Alkoxyalkadiens durch Umsetzung des konjugierten Alkadiens mit dem Alkanol in Gegenwart einer katalytisch wirksamen Menge einer Katalysatorverbindung, umfassend

$$[PdR^1] \quad [R^2_xY(R^3Z)_y] \qquad (II)$$

worin $R^1$ eine organische acyclische oder cyclische Carboxylatgruppe mit 1 bis 10 Kohlenstoffatomen ist, $R^2$ eine cyclische oder acyclische Kohlenwasserstoffgruppe mit bis zu 10 Kohlenstoffatomen ist, Y Phosphor, Antimon, Arsen oder Stickstoff ist, x + y die Wertigkeit von Y angibt, worin y gleich oder größer als 1 ist, $R^3$ eine cyclische oder acyclische Kohlenwasserstoffgruppe mit bis zu 6 Kohlenstoffatomen, Benzyl oder Naphthyl ist, Z eine hydrophile Gruppe, gewählt aus $—SO_3Na$, $—NH_2$, —COOH und quaternären Ammoniumgruppen, ist,

wobei die Umsetzung in wesentlicher Abwesenheit von Sauerstoff und Wasser und in Gegenwart eines polaren Lösungsmittels für den Katalysator durchgeführt wird, um ein Telomer zu erhalten, und das Telomer mit einem Kohlenwasserstofflösungsmittel extrahiert wird.

10. Verfahren nach Anspruch 9, worin das konjugierte Alkadien aus Butadien, Isopren, Chlorpren, Piperylen und 1,3-Pentadien gewählt wird.

11. Verfahren nach Anspruch 9 oder 10, worin das Alkanol aus geradkettigen, verzweigten oder cyclischen Verbindungen mit bis zu 10 Kohlenstoffatomen und wenigstens einer Hydroxygruppe gewählt wird.

12. Verfahren nach Anspruch 11, worin das Alkanol Methanol ist.

13. Verfahren nach Anspruch 9, worin $R^1$ eine Phenylgruppe oder eine Niedrigalkylgruppe mit 1 bis 4 Kohlenstoffatomen umfaßt.

14. Verfahren nach einem der Ansprüche 9 bis 13, worin die Umsetzung bei einer Temperatur von 60°C bis 80°C bei autogenen Drucken bis zu etwa 1379 kPa (200 psig) in einer nichtoxidierenden Atmosphäre unter wasserfreien Bedingungen durchgeführt wird.

15. Verfahren nach einem der Ansprüche 9 bis 14, worin das polare Lösungsmittel aus N-substituierten Amiden, Glykolen, Dimethylsulfoxid und Sulfolan gewählt wird und die Kohlenwasserstofflösungsmittel bis zu 10 Kohlenstoffatome enthalten und aus acyclischen oder cyclischen gesättigten Kohlenwasserstoffen gewählt werden.

16. Verfahren nach einem der Ansprüche 9 bis 15, worin der Katalysator einen Komplex von Palladiumacetat und Natriumdiphenylphosphinobenzol-m-sulfonat umfaßt.

**Revendications**

1. Procédé de télomérisation d'un alcadiène conjugué ayant de 4 à 6 atomes de carbone, avec un alcanol, pour préparer un alcoxyalcadiène, ce procédé consistant à faire réagir ledit alcadiène conjugué et ledit alcanol avec une quantité catalytiquement efficace d'un composé de catalyseur comprenant:

$$[PdR^1] \quad [YR^2_y]_2 \qquad (I)$$

où $R^1$ est un groupe carboxylate organique, acyclique ou cyclique, ayant de 1 à 10 atomes de carbone; et $R^2$ est un radical organique à chaîne droite à chaîne ramifiée ou cyclique, ayant de 1 à 10 atomes de carbone; Y représente le phosphore, l'arsenic ou l'antimoine; y est la valence de Y,

où ledit alcanol est utilisé dans une quantité en excès de la quantite stoechiométrique nécessaire pour la télomérisation, le rapport ligand:Pd est maintenu à une valeur de 10:1 à 1:1 pendant la réaction, ladite réaction étant conduite sensiblement en l'absence d'oxygène et d'eau, et en présence d'un solvant organique à point d'ébullition élevé pour le catalyseur, le point d'ébullition dudit solvant étant supérieur au point d'ébullition dudit alcoxyalcadiène; à séparer par distillation ledit alcoxyalcadiène dudit catalyseur sand addition d'eau, et à recycler ledit catalyseur pour la nouvelle télomérisation dudit alcadiène conjugué avec ledit alcanol.

2. Procédé selon la revendication 1, dans lequel ledit alcadiène conjugué est choisi parmi le butadiène l'isoprène, le chloroprène, le pipérylène et le pentadiène-1,3.

3. Procédé selon la revendication 1 ou 2, dans lequel ledit rapport ligand:Pd est maintenu à une valeur allant de 5:1 à 2:1.

4. Procédé selon l'une des revendications 1 à 3, dans lequel ledit alcanol est choisi parmi des composés à chaîne droite, à chaîne ramifiée ou cyclique, ayant de 1 à 10 atomes de carbone et possédant au moins un groupe hydroxyle.

5. Procédé selon la revendication 4, dans lequel ledit alcanol est le méthanol.

6. Procédé selon la revendication 1, dans lequel $R^1$ est un carboxylate à chaîne droite ou à chaîne ramifiée, ayant de 1 à 4 atomes de carbone.

7. Procédé selon la revendication 1, dans lequel $R^2$ est un groupe phényle ou un groupe alkyle ayant de 1 à 4 atomes de carbone.

8. Procédé selon l'une des revendications 1 à 7, dans lequel ladite réaction est conduite à une température allant de 65°C à 75°C à des pressions autogènes allant jusqu'à 1379 kPa (200 livres par pouce carré au manomètre) dans une atmosphère inerte.

9. Procédé de télomérisation d'un alcadiène conjugué ayant de 4 à 6 atomes de carbone avec un alcanol, pour préparer un alcoxyalcadiène, ce procédé consistant à faire réagir ledit alcadiène conjugué avec ledit alcanol en présence d'une quantité catalytiquement efficace d'un composé catalyseur consistant en:

$$[PdR^1] \quad [R^2_xY(R^3Z)_y] \qquad (II)$$

dans lequel $R^1$ est un groupe carboxylate organique, acyclique ou cyclique, ayant de 1 à 10 atomes de carbone; $R^2$ est un groupe hydrocarboné, cyclique ou acyclique, ayant jusqu'à 10 atomes de carbone; Y représente le phosphore, l'antimoine, l'arsenic ou l'azote; X + y représente la valence de Y, Y étant égal ou supérieur à 1; $R^3$ est un groupe hydrocarboné, cyclique ou acyclique, ayant jusqu'à 6 atomes de carbone, un groupe benzyle ou un groupe naphtyle; Z est un groupe hydrophile choisi parmi les groupes $-SO_3Na$, $-NH_2$, $-COOH$ et ammonium quaternaire,
ladite réaction étant conduite sensiblement en l'absence d'oxygène et d'eau, et en présence d'un solvant polaire pour le catalyseur, pour obtenir un télomère, et à extraire ledit télomère par un solvant hydrocarboné.

10. Procédé selon la revendication 9, dans lequel ledit alcadiène conjugué est choisi parmi le butadiène, l'isoprène, le chloroprène, le pipérylène et le pentadiène-1,3.

11. Procédé selon la revendication 9 ou 10, dans lequel ledit alcanol est choisi parmi les composés à chaîne droite, à chaîne ramifiée ou cycliques, ayant jusqu'à 10 atomes de carbone et au moins un groupe hydroxyle.

12. Procédé selon la revendication 11, dans lequel ledit alcanol est le méthanol.

13. Procédé selon la revendication 9, dans lequel $R^1$ comprend un groupe phényle ou un groupe alkyle inférieur ayant de 1 à 4 atomes de carbone.

14. Procédé selon l'une des revendications 9 à 13, dans lequel ladite réaction est conduite à une température allant de 60°C à 80°C, à des pressions autogènes allant jusqu'à environ 1379 kPa (200 livres par pouce carré), dans une atmoksphère non-oxydante, dans des conditions anhydres.

15. Procédé selon l'une des revendications 9 à 14, dans lequel ledit solvant polaire est choisi parmi les amides N-substitués, les glycols, le diméthylsulfoxyde et le Sulfolane, et lesdits solvants hydrocarbonés contiennent jusqu'à 10 atomes de carbone et sont choisis parmi les hydrocarbures saturés, acycliques ou cycliques.

16. Procédé selon l'une des revendications 9 à 15, dans lequel ledit catalyseur comprend un complexe d'acétate de palladium et de diméthylphosphinobenzène-m-sulfonate de sodium.